# EUROPEAN PATENT APPLICATION

(11) **EP 4 353 816 A1**
(43) Date of publication of application: **17.04.2024**
(21) Application number: 22200854.2
(22) Date of filing: 11.10.2022
(51) Int. Cl.: C12N 5/077, A61K 35/34

(54) **METHODS FOR PRODUCING SINOATRIAL NODE SUBPOPULATIONS**

(71) Applicant: Stichting Amsterdam UMC, 1081 HV Amsterdam (NL)
(72) Inventor: The designation of the inventor has not yet been filed
(74) Representative: de Pauw, Elmar Sebastian David

(57) **Abstract**

The invention relates to a method of generating a population of cardiac progenitor cells from MESP1 mesoderm cells comprising:
a) incubating a MESP1 mesoderm cell in a late mesoderm induction medium comprising a WNT inhibitor for a sufficient period of time to generate a ROR1+ and ROR2+ mesoderm cell,
b) incubating said ROR1+ and ROR2+ mesoderm cell in a cardiac progenitor induction medium comprising a BMP ligand, a WNT inhibitor, retinoic acid, a TGF-β inhibitor or TGF-β, and an FGF inhibitor for a sufficient period of time to generate a cardiac progenitor cell expressing TBX5 and TBX18.

## Description

### FIELD

The present disclosure provides compositions and methods for generating cells with SAN-TZ or SAN-tail characteristics from pluripotent stem cells or mesoderm cells.

### BACKGROUND

The sinoatrial node (SAN) is the primary pacemaker of the heart. Within the cardiomyocyte fraction of the SAN, there are distinct subpopulations such as head, tail and transitional cells (Komosa et al, 2021 Circ Arrhythm Electrophysiol 14: e00995). The pacemaker cells in the SAN-head population express the T-box transcription factors Tbx18 and Tbx3. This region is also distinct from all other cardiomyocytes in the heart as it lacks the expression of Nkx2-5 (Wiese et al, 2009, Circulation Research 104: 388-397). The SAN-tail located inferior to the SAN-head expresses Tbx3 and Nkx2-5 but is devoid of Tbx18 (Wiese et al, , Circulation Research 104: 388-397 2009; Goodyer et al, 2019 Circ Res 125: 379-397). Furthermore, transitional cells (SAN-TZ) with transcriptional and functional properties intermediate to that of pacemaker cells and the adjacent atrial myocardium have also been reported, which are believed to play a critical role in transmitting the electrical impulses from the SAN to the atrial myocardium. Neither SAN-TZ or SAN-tail cells or cells with the characteristics of such cells have been isolated or cultured.

Currently, no methods exist which are capable of generating cells from precursor cells with SAN-TZ or SAN-tail characteristics so that effects of various therapeutics on SAN function or application as cell-based biological pacemakers can be investigated. Biological pacemakers would ideally contain a relatively similar level of heterogeneous nodal cell types, as seen in the SAN. Many studies of the SAN have demonstrated the importance of its transitional cells; however, previous attempts to form biological pacemakers have neglected this important feature of the SAN. Thus, in addition to being able to generate a population of pacing cells, it is likely there will also be a need to generate transitional cells as well.

Dysfunction of SAN cells due to aging or genetic defects result in life-threatening bradycardias. There is a pressing need not only to study and model SAN associated diseases, but also to identify drugs, gene or cell therapies to restore function of the SAN.

It is an object of the current invention to provide a culturing method which enables the generation of precursor cells which can differentiate into SAN-like cells which have SAN-TZ or SAN tail characteristics.

It is also an object of the current invention to provide a culturing method which enables the generation of SAN-like cells which have SAN-TZ or SAN tail characteristics.

It is also an object of the current invention to provide a culturing method which enables the generation of a population of SAN-like cells which are enriched for cells having SAN-TZ characteristics.

It is also an object of the current invention to provide a culturing method which enables the generation of a mixed population of SAN-like cells containing subpopulations having SAN-head, SAN-tail and SAN-TZ characteristics.

It is an object of the invention to provide SAN-like cells which have SAN-TZ characteristics.

It is an object of the invention to provide SAN-like cells which have SAN-tail characteristics.

It is also an object of the current invention to provide a method of testing for drug candidates that can mitigate drug induced cardiac toxicity in cells having SAN-TZ or SAN-tail characteristics.

These and other objects are solved with the methods of the invention disclosed herein.

### SUMMARY

The present disclosure provides compositions and methods for generating cells with SAN-TZ or SAN-tail characteristics from pluripotent stem cells or mesoderm cells.

The invention provides a method of generating a population of cardiac progenitor cells from MESP1 mesoderm cells comprising:
a) incubating a MESP1 mesoderm cell in a late mesoderm induction medium comprising a WNT inhibitor for a sufficient period of time to generate a ROR1+ and ROR2+ mesoderm cell,
b) incubating said ROR1+ and ROR2+ mesoderm cell in a cardiac progenitor induction medium comprising a BMP ligand, a WNT inhibitor, retinoic acid, a TGF-β inhibitor or TGF-β, and an FGF inhibitor for a sufficient period of time to generate a cardiac progenitor cell expressing TBX5 and TBX18. Preferably, said period of time to generate a ROR1+ and ROR2+ mesoderm cell is between 12 and 48 hours, more preferably at least 24 hours. Preferably, said period of time to generate a cardiac progenitor cell expressing TBX5 and TBX18 is between 24 and 96 hours, and more preferably at least 48 hours. These cardiac progenitor cells are the precursor cells which develop characteristics of SAN-TZ cells when cultured further for around 2 - 14 days, optionally at least 2 days, 4 days, 7 days, preferably around 12 days.

In an embodiment, said ROR1+ and ROR2+ mesoderm cell is incubated in a medium comprising a BMP ligand, a WNT inhibitor, retinoic acid, TGF-β. An advantage of this is that it results in an enrichment of SAN-TZ cells.

In another embodiment, said ROR1+ and ROR2+ mesoderm cell is incubated in a medium comprising a BMP ligand, a WNT inhibitor, retinoic acid, TGF-β inhibitor. An advantage of this method is that it results in all three subpopulations of SAN cells, i.e., SAN head, SAN tail and SAN TZ cells.

Preferably, said MESP1 mesoderm cell is prepared by incubating a pluripotent stem cell (PSC) in a medium comprising an activator of WNT signaling and optionally one or more members selected from a BMP ligand, a nodal signaling protein for a sufficient period of time to generate MESP1 mesoderm cells expressing EOMES, MESP1 and MESP2. Preferably, said period of time to generate MESP1 mesoderm cells expressing EOMES is between 24 and 72 hours, Optionally, at least 36, 48 hours, preferably about 72 hours.

The invention further provides a method for generating a population of Sinoatrial node (SAN)-like cells having SAN-head characteristics from MESP1 mesoderm cells, wherein a cardiac progenitor cell is produced by incubating said ROR1+ and ROR2+ mesoderm cell in a cardiac progenitor induction medium which comprises a BMP ligand, a WNT inhibitor, retinoic acid, a TGF-β inhibitor, said method further comprising incubating said cardiac progenitor cell in a SAN induction medium comprising a WNT inhibitor for a sufficient period of time to generate a population of SAN-like cells which express SHOX2, TBX18 and FLRT3 and do not substantially express NKX2-5 and NPPA. In an embodiment, said WNT inhibitor is added to said SAN induction medium after a period of a few days, for example after 1, 2, 3, 4, 5, 6, 7, 8 9, 10 days.

The invention further provides a method for generating a heterogeneous population of Sinoatrial node (SAN)-like comprising subpopulations of SAN-like cells having SAN-head, SAN-tail and SAN-TZ characteristics from MESP1 mesoderm cells, wherein a cardiac progenitor cell is produced by incubating said ROR1+ and ROR2+ mesoderm cell in a cardiac progenitor induction medium which comprises a BMP ligand, a WNT inhibitor, retinoic acid, a TGF-β inhibitor, said method further comprising incubating said cardiac progenitor cell in a SAN induction medium without a WNT inhibitor for a sufficient period of time to generate a population of SAN-like cells, wherein:
- said SAN-like cells having SAN-tail characteristics express ISL1, SHOX2, TBX3 and NKX2-5 and do not substantially express TBX18 and NPPA,
- said SAN-like cells having SAN-head characteristics express SHOX2, TBX18 and FLRT3 and do not substantially express NKX2-5 and NPPA, and
- said SAN-like cells having SAN-TZ characteristics express NPPA, NKX2.5 and TBX3 and do not substantially express TBX18 and SHOX2.

The invention further provides a method for generating a population of SAN-like cells having SAN-TZ characteristics from MESP1 mesoderm cells, wherein a cardiac progenitor cell is produced according to the method according to the invention, and wherein said cardiac progenitor cell is further cultured in a SAN induction medium without a WNT inhibitor for a sufficient period of time to generate a population of SAN-like cells, wherein at least a proportion of SAN-like cells express NPPA, NKX2.5 and TBX3 and do not substantially express TBX18 and SHOX2.

Preferably, said period of time to generate the population of SAN-like cells as referred to in any of the above methods is between 5 and 20 days, preferably between 5 and 10, between 7 and 13, or between 9 and 15 days, preferably at least 5, 6, 7, 8, 9, 10 days. Preferably at most 90, 60 days, 30 days, 20 days.

Further provided is a method for generating a population of SAN-like cells, wherein a cardiac progenitor cell is produced according to the method as described above, and wherein said cardiac progenitor cell is further cultured for a sufficient period of time to generate a population of SAN-like cells, wherein at least a proportion of SAN-like cells express NKX2.5 and TBX3 and do not substantially express TBX18. Preferably said proportion is at least 5, 10, 20 or 25% of the total population.

In a preferred embodiment, the SAN-like cells exhibit electrophysiological properties of SAN cells. Preferably, the electrophysiological properties exhibited by SAN-like cells comprise exhibition of HCN dependent funny current, *I_{f}.*

In a preferred embodiment of the methods of the invention described above, said late mesoderm induction medium comprises WNT inhibitor in a concentration between 250 nM -10 µM. Preferably, said cardiac progenitor induction medium comprises 0.1 - 2 µM retinoic acid, 0.25 -10 µM of a WNT inhibitor, 0.1 - 1 µM of an FGF inhibitor, 1-20 ng/mL TGF-β, 0.1 µM - 10 TGF-β inhibitor and/or 1-20 ng/mL of a BMP ligand.

The invention further provides A method of identifying a candidate drug comprising:
a. generating a population of SAN-like cells according to the method of the invention;
b. contacting the SAN-like cells with a candidate test drug;
c. measuring the beat rate, action potential characteristics and/or ion currents of the SAN-like cells;
d. comparing the beat rate, the action potential characteristics and/or ion currents of the SAN-like cells to a control SAN-like cells not treated with the candidate test drug; and
e. selecting the candidate test drug which modulates the beat rate and/or action potential compared to the control cell as the candidate drug.

Preferably, said population of cells comprises at least 5, 10, or 20% of SAN-like cells which express NPPA, NKX2.5 and TBX3 and do not substantially express TBX18 and SHOX2 or said population of cells comprises at least at least 5, 10, or 20% of SAN-like cells which express NKX2-5, SHOX2, TBX3, TBX3, ISL1 and do not substantially express TBX18.

The invention further provides an isolated SAN cell or a SAN-like cell which has the following characteristics:
- expresses NPPA, NKX2.5 and TBX3 and does not substantially express TBX18 and SHOX2, or
- expresses NKX2-5, SHOX2, TBX3, TBX3, ISL1 and does not substantially express TBX18.

The invention further provides a SAN-like cell obtainable by the method according to the invention.

The invention further provides the isolated SAN cell or SAN-like cell of the invention, for use in a medical treatment of a patient, preferably for the treatment of bradycardia or nodal dysfunction.

The invention further provides the use of the isolated SAN cell or SAN-like cells obtained by the method according to the invention in a drug discovery method.

### BRIEF DESCRIPTION OF THE FIGURES

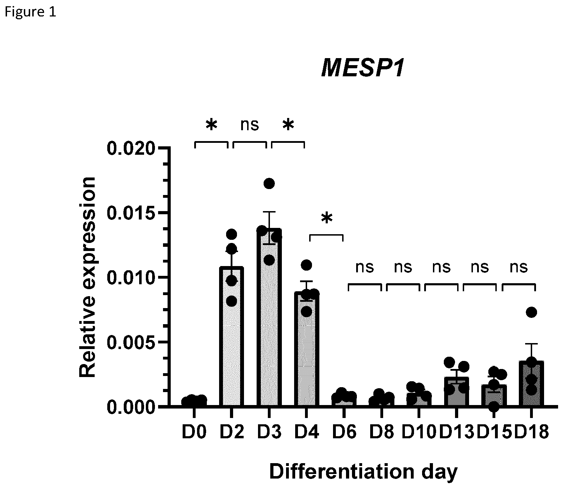

Figure 1 shows the expression of *MESP1* as measured by RT q-PCR. Differentiation was induced on day 0 using early mesoderm induction medium. On day 3, cells were incubated with late mesoderm induction medium. On day 4, cells were incubated with cardiac progenitor induction medium. For RT q-PCR, cells were collected on day 0, day 2, day 3, day 4, day 6, day 8, day 10, day 13, day 15 and day 18 of the differentiation process. RNA isolation, cDNA synthesis and RT-qPCR were performed as described in the methods. Relative expression was obtained by normalizing expression of target genes to housekeeping genes *RPLP0* and *GUSB.* As depicted in the figure, noticeable expression of *MESP1* begins on day 2 of differentiation. Expression peaks on day 3 and starts to decline again by day 4 of differentiation. By day 6, *MESP1* has expression has returned to baseline levels on day 0. ^{∗}*P*<0.05 was considered significant using Mann-Whitney test.

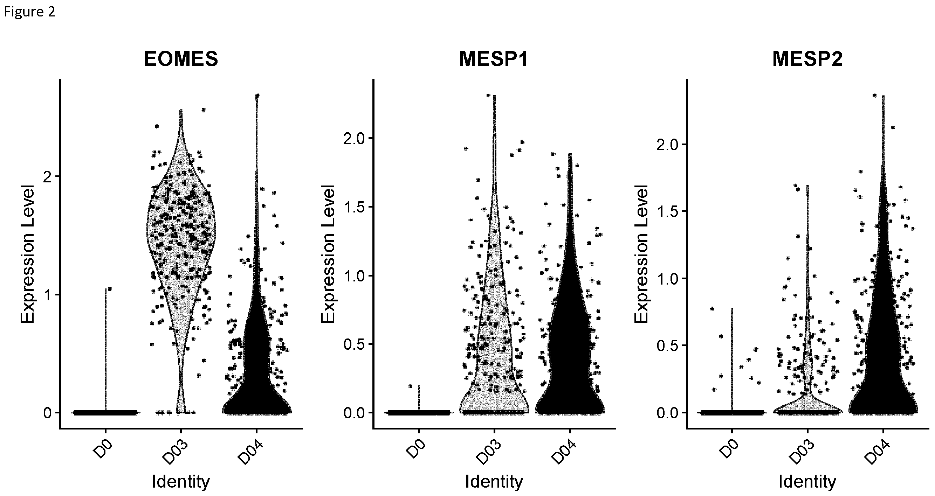

Figure 2 shows a violin plots depicting expression of *EOMES, MESP1*, and *MESP2* on day 0, day 3 and day 4 of differentiation as determined by single cell RNA sequencing.

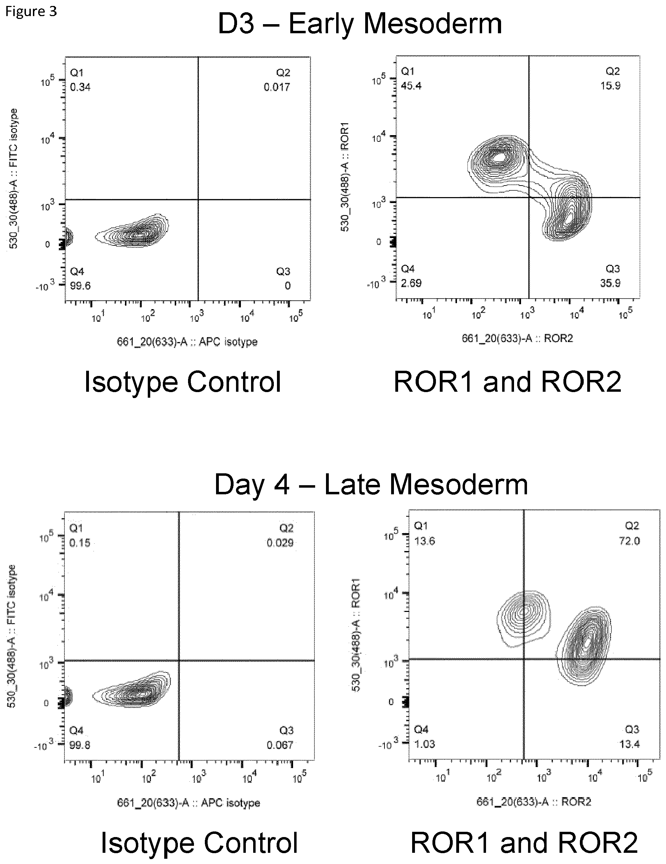

Figure 3 shows the expression of ROR1 and ROR2 on day 3 (top right, panel) and day 4 (bottom, right panel) as determined by flow cytometry. Differentiation was induced on day 0 using early mesoderm induction medium. On day 3, cells were incubated with late mesoderm induction medium. Flow cytometry was performed on day 3 and day 4 using antibodies against ROR1 or ROR2. A matching isotype control was used as negative control for each experiment (top left panel and bottom left panel). In the contour plots depicted in the figure, ROR1 expression is displayed on Y-axis and ROR2 expression on the X-axis. At the early mesoderm stage on day 3, a large majority of cells express either ROR1 (45.4%) or ROR2 (35.09%). At the late mesoderm stage on day 4, a large majority of cells (72%) express both ROR1 and ROR2.

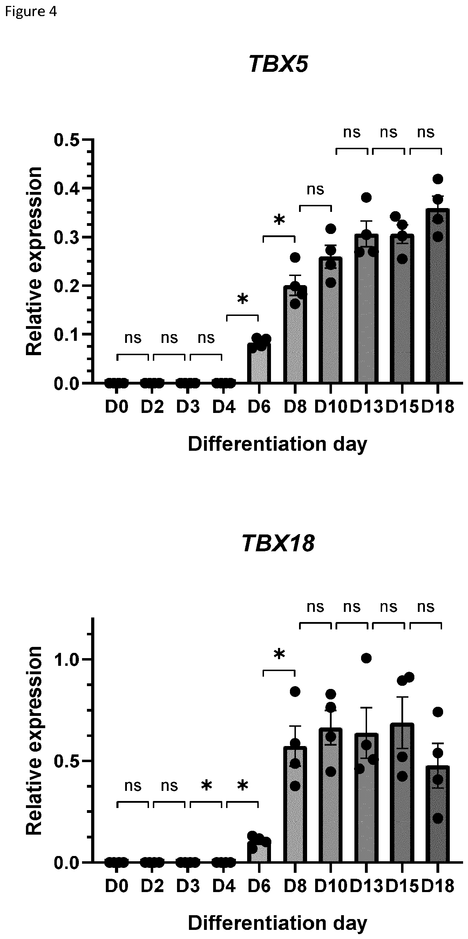

Figure 4 shows the expression of *TBX5* and *TEX18* as determined by RT q-PCR. Differentiation was induced on day 0 using early mesoderm induction medium. On day 3, cells were incubated with late mesoderm induction medium. On day 4, cells were incubated with cardiac progenitor induction medium. For RT q-PCR, cells were collected on day 0, day 2, day 3, day 4, day 6, day 8, day 10, day 13, day 15 and day 18 of the differentiation process. RNA isolation, cDNA synthesis and RT-qPCR were performed as described in the methods. Relative expression was obtained by normalizing expression of target genes to housekeeping genes *RPLP0* and *GUSB.* As depicted in the figure, *TBX5* and *TEX18* expression is first noticeable at day 6. Expression further increases on day 8 and is maintained thereafter. ^{∗}*P*<0.05 was considered significant using Mann-Whitney test.

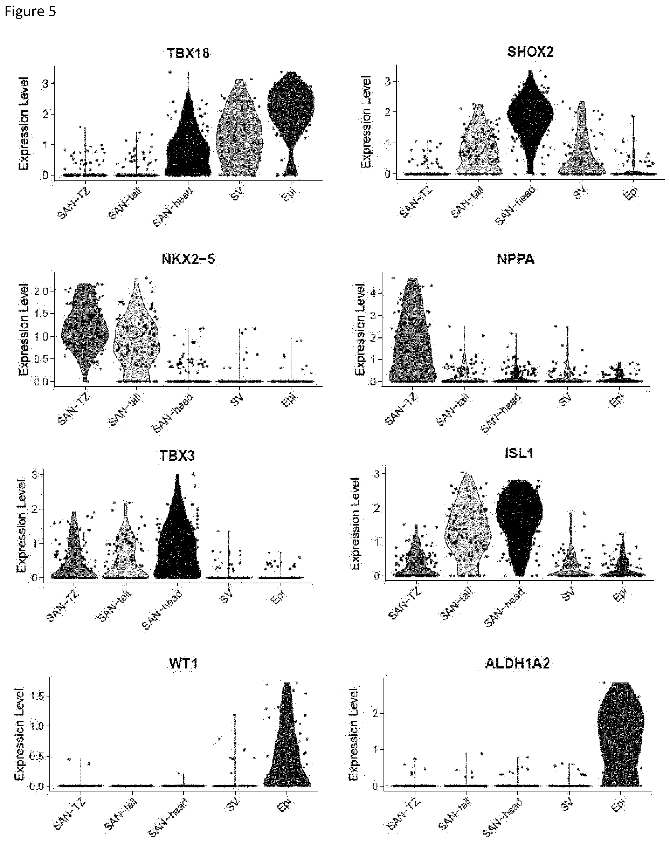

Figure 5 shows violin plots depicting expression of *TBX 18*, *SHOX2*, *NKX-5*, *NPPA*, *TBX3, ISL1*, *WT1* and *ALDH1A2* that mark SAN subpopulations such as head (SAN-head), tail (SAN-tail) and transitional (SAN-TZ) cells as well as side populations of sinus venosus (SV) and proepicardial cells (Epi).

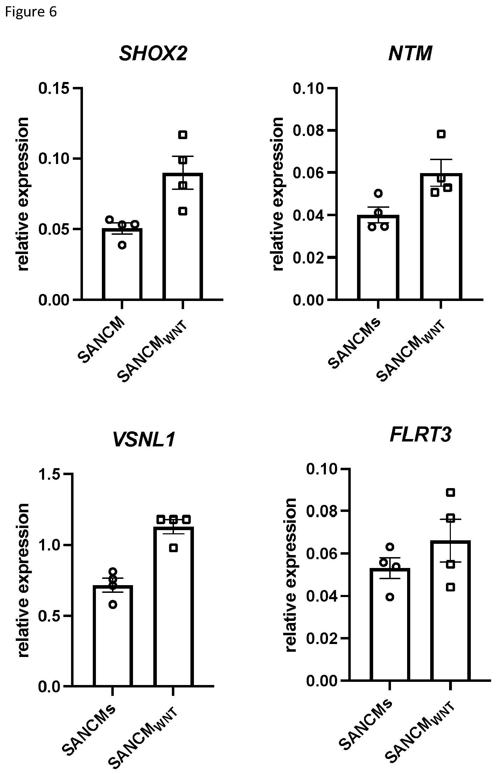

Figure 6 shows Relative expression of SAN-head associated genes by RT q-PCR in cell populations obtained in Example 1 (SANCM) and Example 2 (SANCM_{WNT}). RNA isolation, cDNA synthesis and RT-qPCR were performed as described in the methods. Relative expression was obtained by normalizing expression of target genes to housekeeping genes *RPLP0* and *GUSB.*

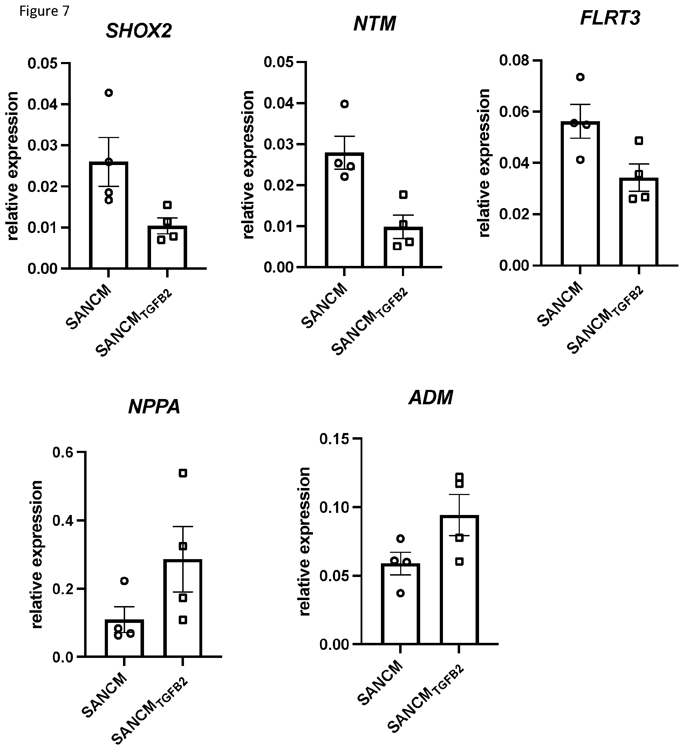

Figure 7 shows Relative expression of SAN-head (*SHOX2*, *NTM*, *FLRT3*) and SAN-TZ (*NPPA, ADM*) by RT q-PCR in cell populations obtained in Example 1 (SANCM) and Example 3 (SANCM_{TGFB2}). RNA isolation, cDNA synthesis and RT-qPCR were performed as described in the methods. Relative expression was obtained by normalizing expression of target genes to housekeeping genes *RPLP0* and *GUSB.*

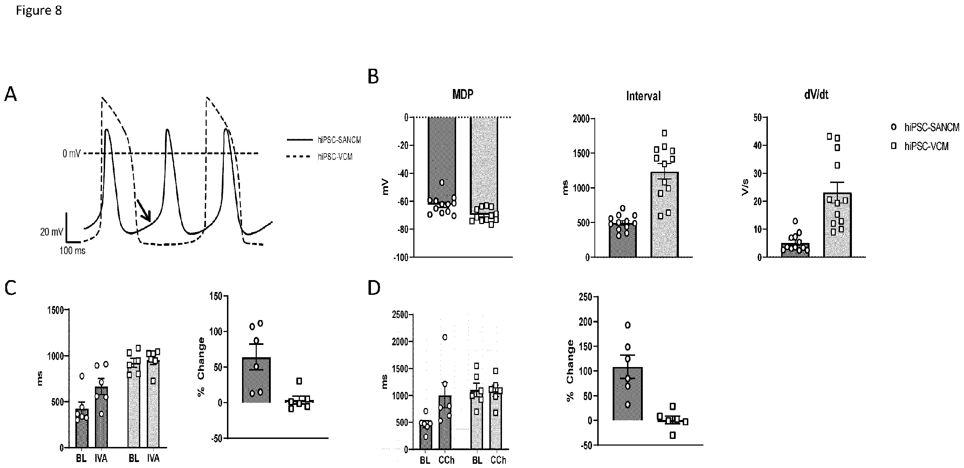

Figure 8 shows the electrophysiological characterization of hiPSC-derived SAN-like (SANCM) and ventricular-like cardiomyocytes (VCM). (A) Action potential traces demonstrate a prominent diastolic depolarization phase in SANCM. (B) Quantified data shows less negative maximum diastolic depolarization (MDP), faster interval and slower upstroke velocity (dV/dt) in SANCM. (C) In response to ivabradine (Iva) and carbachol (CCh), the beating rate of SAN-like cardiomyocytes slows down whereas there is no effect in ventricular-like cardiomyocytes.

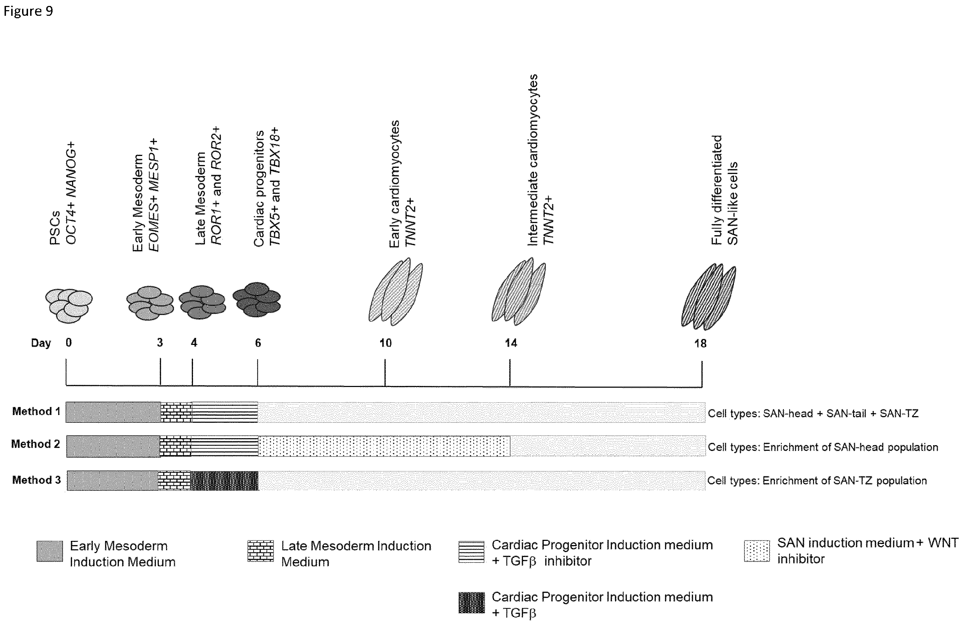

Figure 9 is a schematical overview of the three different culturing methods according to the invention which result in SAN-like cells.

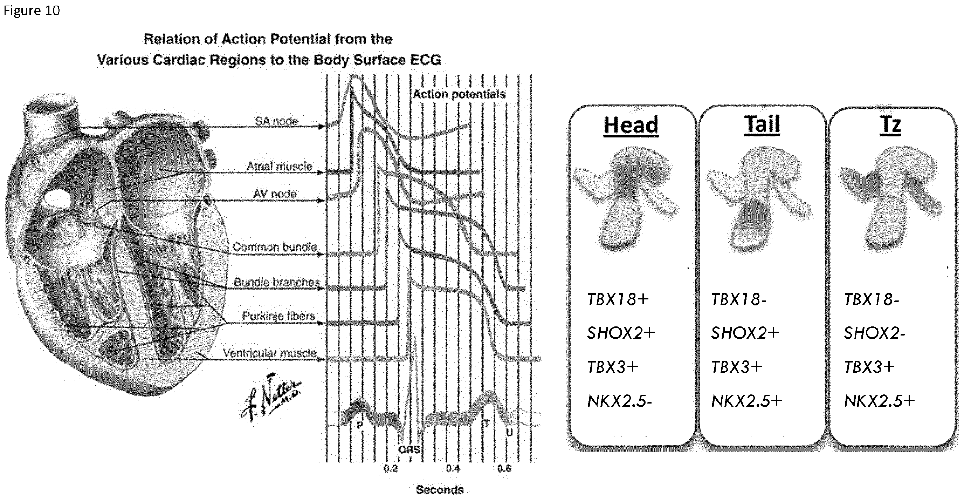

Figure 10 shows the sinoatrial node (SAN), the primary pacemaker of the heart (left). The SAN initiates electrical impulses and maintains heart rate and rhythm. Dysfunction due to aging or genetic defects result in life-threatening bradycardias. Within the SAN, three distinct subpopulations of SAN cells are present, i.e. head, tail and transitional cells.

### DETAILED DESCRIPTION

### Definitions

The term "not substantially express" as used herein refers to an expression level that is regarded a background level which can be found in cells of a negative control population. For instance, for a population of cells expressing TBX18 and SHOX2, a suitable negative control population is a population containing ventricular cardiomyocytes derived from pluripotent stem cells. Ventricular cells are preferably cells which express MYL2 and/or MYH7, HEY2, IRX4, HOPX.

The terms "express" and "expression level" as used herein may refer to expression of genes or proteins.

The term "cardiac progenitor cell" as used herein refers to a progenitor cell that is committed to the cardiac lineage and that has the capacity to differentiate into all three cardiac lineage cells (cardiac muscle cells, endothelial cells and smooth muscle cells). Preferably, said cardiac progenitor cell is a TBX18+ progenitor cell which can give rise to myocardium, epicardium and epicardial derivatives such as smooth muscle cells, fibroblasts etc. Preferably, it also expresses TBX5.

The term "MESP1 mesoderm cells" as used herein refers to cells which may be produced from pluripotent stem cells (PSC) and have the following characteristics:
- express MESP1, as detected by quantitative real-time PCR as described herein, and preferably also MESP2.
- express NANOG at a lower level compared to PSC and preferably express OCT4 at a lower level than PSC after 3 days of culturing in a late mesoderm induction medium
- can differentiate into cardiomyocytes

The term "WNT inhibitor" as used herein means any agent, including any compound and/or protein that inhibits WNT signaling, including but not limited to WNT antagonists that bind either to the WNT ligand itself, or to WNT receptors, such as Dickkopf (Dkk) proteins, WNT Inhibitory Factor-1 (WIF-1), and secreted Frizzled-Related Proteins (sFRPs), as well as WNT inverse agonists (e.g. an agent that binds to the same receptor as an agonist but induces a pharmacological response opposite to that of an agonist). Examples of WNT inhibitors include XAV939, IWP 2, an inhibitor of wnt processing, and iCRT14, which is a potent inhibitor of β-catenin-responsive transcription (CRT), both of which are available from Tocris Bioscience, as well as combinations thereof.

The term "activator of WNT signaling" as used herein means any agent, including any compound and/or protein that stimulates WNT signaling, including but not limited to CHIR 99021, BIO, LY2090314, DCA.

The term "ROR1+ and ROR2+ mesoderm cell" as used herein refers to a cell having the following characteristics:
- express ROR1 and ROR2, as detected by flow cytometry as described herein
- can differentiate into cardiomyocytes

The term "BMP ligand" as used herein means any molecule optionally any BMP or growth and differentiation factor (GDF) that activates the type I and type II serine-threonine kinase receptors including BMPRIA (ALK3), BMPR1B (ALK6), including for example BMP4 and BMP2.

The term "BMP4" (for example Gene ID: 652) as used herein refers to Bone Morphogenetic Protein 4, for example human BMP4, as well as active conjugates and fragments thereof, that can for example activate BMP4 receptor signaling.

The term "retinoic acid" or "RA" includes vitamin A and metabolites of vitamin A that mediate the function of vitamin A, and includes for example all-trans RA (e.g. Sigma R2625), 9-cis RA (e.g. Sigma R4643), and retinol (e.g. Sigma R7632) as well as RA analogs (e.g. RAR agonists), such as AM580, a selective RARα agonist (Tocris 0760), AC55649, a selective RARβ agonist (Tocris 2436), and CD437, a selective RARγ agonist (Tocris 1549). The term "FGF inhibitor" as used herein means any FGF receptor inhibitor (FGFR 1,2,3,4) or FGF signaling inhibitor (i.e. downstream p38 MAPK inhibitor), including but not limited to PD 173074 (Tocris) and SU 5402 (Tocris) and p38 MAPK inhibitor SB203580 (Tocris).

The term "TGF-β inhibitor" as used herein means any molecule that inhibits signal of the TGF-β, including but not limited to SB431542 (Tocris) and LY364947 (Tocris).

The term "TBX5" as used herein refers to the expression product of the T-box transcription factor 5 gene.

The term "TBX18" as used herein refers to the expression product of the T-box transcription factor 18 gene.

The term "pluripotent stem cell" (also referred to as "PSC") as used herein refers to a cell having an having an ability to differentiate to derivatives of all three germ layers, i.e., ectoderm, mesoderm and endoderm and also having self-renewal capacity which is an ability to maintain the pluripotency during cell division. "PSCs" include Embryonic Stem Cells (ESCs), which are derived from inner cell mass of blastocysts or morulae, including cells that have been serially passaged as cell lines. Embryonic stem cells, regardless of their source or the particular method used to produce them, can be identified based on their ability to differentiate into cells of all three germ layers, expression of at least Oct4 and alkaline phosphatase, and ability to produce teratomas when transplanted into immunodeficient animals. The term PSCs also includes induced PSCs (iPSCs), which are cells converted from somatic cells by a variety of methods, such as a transient overexpression of a set of transcription factors. A PSC may be a cell of any species with no limitation, and preferably a mammalian cell. It may be a rodent or primate cell. For example, it may be a monkey, mouse or a human pluripotent stem cell. The term "human pluripotent stem cells" or hPSCs includes human embryonic stem cells and human induced PSCs. Human embryonic stem cells may be obtained from established lines of human embryonic stem cells or human embryonic germ cells, such as, for example the human embryonic stem cell lines HI, H7, and H9 (WiCell).

The term "sinoatrial node-like cells" or "SAN-like cells" as used herein refers to cardiomyocytes or cardiac cells that express one or more of sinoatrial nodal (SAN) cell markers TBX18, TBX3, NKX2-5, SHOX2 and ISL1 and which are capable of displaying pacemaker activity (e.g., display "funny current"). The SAN cells can initiate beating in certain conditions, and they display a "funny current", which is a mixed sodium- potassium current that activates upon hyperpolarization at voltages in the diastolic range (normally from -60/-70 mV to -40 mV). The funny current flows through an HCN-dependent channel. Unlike atrial cardiomyocytes, they do not express Cx40 in vivo. Notable electrophysiological properties of SAN-like head cells in comparison with ventricular-like cardiomyocytes are a) a less negative maximum diastolic depolarization (MDP), b) faster interval and c) slower upstroke velocity. Furthermore, similar to in vivo SAN cells, SAN-like cells in vitro show responses to ivabradine, which targets funny current, *I*_{f} and carbachol, which activates inward rectifier potassium current, *I*_{KACH} (see Figure 8).

Within the cardiomyocyte fraction of the SAN, there are distinct subpopulations such as head, tail and transitional cells. The pacemaker cells in the SAN-head population express the T-box transcription factors TBX18, TBX3 as well as SHOX2 and ISL1 (see Figure 5). This region is also distinct from all other cardiomyocytes in the heart as it lacks the expression of Nkx2-5. The SAN-tail is located inferior to the SAN-head. SAN tail cells express ISL1, SHOX2, TBX3 and NKX2-5 but are devoid of TBX18 (Wiese et al, 2009; Goodyer et al, 2019 and Figure 5).

The term "sinoatrial node transitional cells" (SAN-TZ) as used herein refers to cells having the following characteristics:
- express TBX3, NKX2-5 and NPPA when detected as described herein
- do not substantially express TBX18 and SHOX2

The term "SAN-like cells" preferably refers to cells having the expression characteristics of SAN-tail, SAN-head or SAN-TZ as described above.

The term "sinoatrial node cells" or "SAN cells" as used herein refers to cells isolated from a sinoatrial node.

The term "cardiomyocyte" as used herein is a cardiac lineage cell that expresses cTNT and ACTN2. Mesoderm cells, cardiomyocytes, cardiac progenitor cells, SAN cells and SAN-like cells according to the invention are preferably human.

The term "incubating" as used herein includes any in vitro method of maintaining and/or propagating a population of cells, including monolayer, bead, flask, or 3D cultures, optionally where ambient conditions are controlled as in an incubator and optionally involving passaging of cells. Steps that involve Incubating the cells with one or more components, the components can be added simultaneously, at different times, for overlapping periods or for distinct periods. A factor can be added to the medium after the cells have started incubating in for example an induction medium or the factor can be added to the medium before the medium is added to the cells. Further, cells may be washed between incubations, for example to reduce the level of a component from a previous incubation.

The term "culturing" as used herein means any in vitro method of maintaining and propagating a population of cells at least through one cell division, including monolayer, bead, flask, or 3D cultures, optionally where ambient conditions are controlled as in an incubator.

The term "FGF inhibitor" as used herein means any FGF receptor inhibitor (FGFR1,2,3,4) or FGF signaling inhibitor (i.e. downstream p38 MAPK inhibitor), including but not limited to PD173074 (Tocris) and SU 5402 (Tocris) and p38 MAPK inhibitor SB203580 (Tocris).

The term " nodal signaling protein " as used herein refers to any molecule that activates Activin/Nodal signaling. Preferably, said molecule is Activin-A.

The term "medium" as used herein refers to a culturing medium that is suitable for culturing animal cells, optionally supplemented with factors.

The term "early mesoderm induction medium as used herein refers to a medium suitable for allowing a pluripotent stem cell to differentiate into a MESP1 mesoderm cell,

The term "late mesoderm induction medium" as used herein refers to a medium suitable for allowing a MESP1 mesoderm cell to differentiate into a ROR1+ and ROR2+ mesoderm cell.

The term "cardiac progenitor induction medium" as used herein refers to a medium suitable for allowing a ROR1+ and ROR2+ mesoderm cell to differentiate into a cardiac progenitor cell expressing TBX5 and TBX18.

The term "SAN induction medium" as used herein refers to a medium suitable for allowing a cardiac progenitor cell expressing TBX5 and TBX18 to differentiate into a SAN-like cell.

### Description of the embodiments

The present disclosure provides compositions and methods for generating cells with SAN-TZ or SAN-tail characteristics from mesoderm cells or pluripotent stem cells. The methods herein described have an advantage over existing methods (for example as disclosed in WO2021113382A1) as these methods do not result in cells with SAN-tail and SAN-TZ characteristics. The inventor has found that a culturing method comprising steps of incubating a MESP1 mesoderm cell in a late mesoderm induction medium comprising a WNT inhibitor for a sufficient period of time to generate a ROR1+ and ROR2+ mesoderm cell, and subsequently incubating said ROR1+ and ROR2+ mesoderm cell in a cardiac progenitor induction medium comprising a BMP ligand, a WNT inhibitor, retinoic acid, a TGF-β inhibitor or TGF-β, and an FGF inhibitor for a sufficient period of time, result in cardiac progenitor cells which have a unique capability of differentiating into SAN-like cells which have TZ, tail and/or TZ characteristics. Depending on subsequent a culturing step, these cardiac progenitor cells differentiate in all three mentioned SAN-like cells, or to enriched populations of SAN-like cells.

The invention provides a method of generating a population of cardiac progenitor cells from MESP1 mesoderm cells comprising:
a) incubating a MESP1 mesoderm cell in a late mesoderm induction medium comprising a WNT inhibitor for a sufficient period of time to generate a ROR1+ and ROR2+ mesoderm cell,
b) incubating said ROR1+ and ROR2+ mesoderm cell in a cardiac progenitor induction medium comprising a BMP ligand, a WNT inhibitor, retinoic acid, a TGF-β inhibitor or TGF-β, and an FGF inhibitor for a sufficient period of time to generate a cardiac progenitor cell expressing TBX5 and TBX18.

Incubation conditions for cell cultures are known in the art. For example, the conditions typically include culturing at a temperature of between about 32-40 °C, for example, at least or about 32, 33, 34, 35, 36, 37, 38, 39 or 40 °C, preferably at 37 °C. The CO₂ concentration is generally about 1 to 10%, for example, about 2 to 7%, or about 5% or any range or value between 1 and 10%. The oxygen tension is adjusted to generally to provide normoxic conditions and is preferably about 20%.

The cells, starting with the MESP1 mesoderm cells are cultured in late mesoderm induction medium comprising a WNT inhibitor for a sufficient period of time to generate a ROR1+ and ROR2+ mesoderm cell. Said late mesoderm induction medium may be any medium that is routinely used for culturing animal cells can be used, except that no growth factors or serum should be present or are added in the media. Examples of suitable culture media include APEL medium, BPEL medium, BME, F-12, BGJb, MCDB131, CMRL 1066, Glasgow MEM, Improved MEM Zinc Option, IMDM, Medium 199, Eagle MEM, DMEM, Ham, RPMI 1640, and Fischer's media, but other similar media can also be used. Non-growth factor additives, such as antibiotics, B-27 supplement (with or without insulin), amino acids, salts, ascorbic acid and thioglycerol can be added to the media.

In an embodiment, the late mesoderm induction medium comprises a WNT inhibitor at a concentration ranging between about 250 nM-10 µM. Preferably, between 200 nM-8 µM, 300 nM-6 µM

In an embodiment, the late mesoderm induction medium comprises XAV939 at a concentration of about 1 µM, about 2 µM, about 3 µM, about 4 µmol /mL, about 5 µM, about 8 µM, or up to about 10 µM.

The presence of a WNT inhibitor is essential during this phase to steer the MESP1 mesoderm cells toward ROR1+ and ROR2+ mesodermal cells. Preferably the late mesoderm induction medium culturing comprises between 250 nm-10 µM of a WNT inhibitor or a concentration of a WNT which has a comparable effect as XAV939 in said concentration range. Any WNT inhibitor may be used. Preferably, said WNT inhibitor is XAV939. Preferably the late mesoderm induction medium is BPEL medium containing 5 µmol/L XAV939.

Typically, the culturing time is about 24 hours to generate ROR1+ and ROR2+ mesodermal cells from MESP1 mesoderm cells. As demonstrated in Example 1 and in Figure 3, a large majority of the cell fraction expressed both ROR1 and ROR2 within 24 hours. The expression of ROR1 and ROR2 is suitably determined using flow cytometry as described herein. Preferably, said culturing time is at least 15, 16, 17, 18, 19, 20, 21, 22, 23 or 24 hours.

In an embodiment, MESP1 mesoderm cells are incubated with BPEL medium containing 5 µmol/L XAV939 (Tocris Bioscience, #3748/10) to steer the MESP1+ mesodermal cells towards ROR1+ and ROR2+ mesodermal cells for about 24 hours.

In an embodiment, a cell enrichment step is used to further increase the number of said ROR1+ and ROR2+ mesoderm cells. Such methods are well known in the art and include for instance Immunomagnetic cell separation, Fluorescence-activated cell sorting (FACS), Density gradient centrifugation, Immunodensity cell isolation, and Microfluidic cell sorting.

Next, to generate TBX5+ and TBX18+ cardiac progenitor cells that can give rise to sinoatrial node (SAN)-like cells, ROR1+ and ROR2+ mesodermal cells are incubated in a cardiac progenitor induction medium comprising a BMP ligand, a WNT inhibitor, retinoic acid, a TGF-β inhibitor or TGF-β, and an FGF inhibitor. In about 48 hours, cardiac progenitor cells can be detected based on their expression of TBX5 and TBX18, which can be suitably determined by RT-qPCR as described herein.

The main characteristics of these cardiac progenitor cells is the expression of TBX5 and TBX18 as may be determined using quantitative real-time PCR or flow cytometry. Typically, culturing for around 2 days results in cardiac progenitor cell expressing TBX5 and TBX18. The cardiac progenitor cells thus obtained have an advantage in that they are capable of differentiating in 3 different types of SAN-like cells.

The cardiac progenitor induction medium may comprise TGF-β or a TGF-β inhibitor. Either way, the resulting cardiac progenitor cells are capable of producing SAN TZ cells. The difference is however in the number of SAN TZ cell which may be obtained after culturing the cells for a sufficient period of time to generate a population of SAN-like cells. When TGF-β is used in the cardiac progenitor induction medium, then resulting cells express lower levels of *SHOX2*, *NTM,* and *FLRT3* and higher levels of *NPPA*, and *ADM* compared with the expression levels of these genes in cell population obtained in Example 1. This indicates that the use of a TGF-β results in a lower fraction of SAN-head like and a higher fraction of SAN-TZ like cells (see Figure 7).

When a TGF-β inhibitor is used in said cardiac progenitor induction medium, then an increased proportion of SAN-head like cardiomyocytes (see Figure 6).

In an embodiment, said cardiac progenitor induction medium comprises a BMP ligand, a WNT inhibitor, retinoic acid, TGF-β.

In an embodiment, said cardiac progenitor induction medium comprises 0.1 - 2 µM retinoic acid, 0.25 -10 µM of a WNT inhibitor, 0.1 - 1 µM of an FGF inhibitor, 1-20 ng/mL TGF-β inhibitor and/or 1-20 ng/mL of a BMP ligand. Preferably, said cardiac progenitor induction medium comprises 0.1 - 1 µM, 0.15 - 0.8 µM, or 0.2 - 0.6 µM retinoic acid. In an embodiment, said cardiac progenitor induction medium comprises 1 -10 µM, 2 - 8 µM, 3 -7 µM, or 4 - 6 µM of a WNT inhibitor. In an embodiment, said cardiac progenitor induction medium comprises 0.1 - 1 µM, 0.15 - 0.5 µM, or 0.2 - 0.4 µM of an FGF inhibitor. In an embodiment, said cardiac progenitor induction medium comprises 0.1-10, 1-8, 2-6, 3-6 µM TGF-β inhibitor. In an embodiment, said cardiac progenitor induction medium comprises 1-20, 1-20, 1-15, 1-12, 1-10, 1-5, or 2-4 ng/mL of a BMP ligand. In an embodiment, said cardiac progenitor induction medium comprises between 1-20, 2-18, 3-17, or 4-16 ng/mL TGF-β, preferably around 5 ng/mL.

In a preferred embodiment, said cardiac progenitor induction medium comprises BPEL medium containing 2.5 ng/mL BMP4, 5 µmol/L XAV939, 5 µmol/L SB431542 (Tocris, #1614), 250 nmol/L RA (Sigma, #R2625-50MG) and 250 nmol/L PD173074 (Selleck Chemicals, #1264).

In another embodiment, said cardiac progenitor induction medium comprises a BMP ligand, a WNT inhibitor, retinoic acid, and a TGF-β inhibitor.

In another preferred embodiment, said cardiac progenitor induction medium comprises BPEL medium containing 2.5 ng/mL BMP4, 5 µmol/L XAV939, 5 ng/mL TGFB2 (R&D Systems, #302-B2-002/CF), 250 nmol/L RA (Sigma, #R2625-50MG) and 250 nmol/L PD173074 (Selleck Chemicals, #1264).

In an embodiment, a cell enrichment step is used to further increase the number of said cardiac progenitor cells.

Suitable MESP1 mesoderm cells may be generated using methods known in the art. See for example, Wiesinger et al, 2022. A single cell transcriptional roadmap of human pacemaker cell differentiation. eLIFE. 11:e76781 (2022). Preferably, said MESP1 mesoderm cell is prepared by incubating a pluripotent stem cell (PSC) in an early mesoderm induction medium comprising one or more members selected from a BMP ligand, a nodal signaling protein and activators of WNT signaling for a sufficient period of time to generate MESP1 mesoderm cells expressing EOMES and MESP1 and preferably also MESP2. Good results are obtained between 2 and 6 days of culturing in said early mesoderm induction medium. The expression of MESP1 peaks on day 3 and starts to decline again by day 4 of differentiation. By day 6, MESP1 expression typically returns to baseline levels of day 0.

Said early mesoderm induction medium may be any medium that is routinely used for culturing animal cells can be used, except that no growth factors or serum should be present or are added in the media. Examples of suitable culture media include APEL medium, BPEL medium, BME, F-12, BGJb, MCDB131, CMRL 1066, Glasgow MEM, Improved MEM Zinc Option, IMDM, Medium 199, Eagle MEM, DMEM, Ham, RPMI 1640, and Fischer's media, but other similar media can also be used. Non-growth factor additives, such as antibiotics, B-27 supplement (with or without insulin), amino acids, salts, ascorbic acid and thioglycerol can be added to the media. In a preferred embodiment, the early mesoderm induction medium comprises 20 ng/mL Activin-A, 20 ng/mL BMP4, and 1.5 µmol/L CHIR99021. For optimal cardiac differentiation, PSCs are seeded at a density of 2.5-3×10⁴ cells/cm². Differentiation toward early mesoderm was induced when cells reached 80-90% confluency. Preferably, said early mesoderm induction medium comprises BPEL medium (Ng *et al*, 2008) supplemented with 20 ng/mL Activin-A (Miltenyi Biotec, #130-115-012), 20 ng/mL BMP4 (R&D systems, #314-BP-010/CF), and 1.5 µmol/L CHIR99021 (Axon Medchem, #1386) for three days.

Further provided is a method for generating a population of Sinoatrial node (SAN)-like cells from MESP1 mesoderm cells, wherein a cardiac progenitor cell is produced by incubating a ROR1+ and ROR2+ mesoderm cell in a cardiac progenitor induction medium comprising a BMP ligand, a WNT inhibitor, retinoic acid, a TGF-β inhibitor, and an FGF inhibitor for a sufficient period of time to generate a cardiac progenitor cell expressing TBX5 and TBX18. Preferably, said period of time is between 24 - 96 hours, and more preferably at least 48 hours. Subsequently, said cardiac progenitor cell is incubated in a SAN induction medium comprising a WNT inhibitor for a sufficient period of time to generate a population of SAN-like cells which express SHOX2, TBX18 and FLRT3. The use of this method results in enhancing the percentage of SAN-head cells. Typically said sufficient period of time is between 6 and 14 days, more preferably around 10 days.

The invention further provides a method for generating a population of SAN-like cells, wherein a cardiac progenitor cell is produced according to the method of generating a population of cardiac progenitor cells from MESP1 mesoderm cells as described above, and wherein said cardiac progenitor cell is further cultured in a SAN induction medium for a sufficient period of time to generate a population of SAN-like cells, wherein at least a proportion of SAN-like cells express NKX2.5 and TBX3 and do not substantially express TBX18, wherein said SAN induction medium does not contain a WNT inhibitor. Preferably, said SAN induction medium does not contain any growth factors.

In an embodiment, said SAN induction medium is a culture medium refreshed every 2 - 3 days.

In an embodiment, a cell enrichment step is used to further increase the number of said SAN-like cells.

A further aspect includes various uses of the isolated population of SAN like cells according to the invention. Uses include transplantation, for example for in vivo pacemaking as a biological pacemaker, disease modelling and testing candidate drugs. Other uses include studying the safety pharmacology testing of drugs for potential side effects on SAN-like cells and the development of SAN pacemaker unit from PSC, MESP1 mesoderm cells or cardiac progenitor cells, and studying the physiology of human SAN pacemaker as healthy human samples are not readily available.

Accordingly a further aspect is a method of identifying a candidate drug
a. generating SAN-like cells according to a method described herein;
b. contacting the SAN-like cells with a candidate test drug;
c. measuring the beat rate, action potential characteristics and/or ion currents of the SAN-like cells;
d. comparing the beat rate, the action potential characteristics and/or ion currents of the SAN-like cells to a control SAN-like cells not treated with the candidate test drug; and
e. selecting the candidate test drug which modulates the beat rate and/or action potential compared to the control cell as the candidate drug.

In an embodiment, the candidate drug is for treating diseases affecting the SAN, including Sinus Node Dysfunction or Sick Sinus Syndrome (SSS), bradycardia, tachycardia, sinus node arrest/block caused by aging (mostly fibrosis of the sinus node) or electrical remodeling or a genetic disorder.

The above disclosure generally describes the present application. A more complete understanding can be obtained by reference to the following specific examples. These examples are described solely for the purpose of illustration and are not intended to limit the scope of the application. Changes in form and substitution of equivalents are contemplated as circumstances might suggest or render expedient. Although specific terms have been employed herein, such terms are intended in a descriptive sense and not for purposes of limitation.

The following non-limiting examples are illustrative of the present disclosure:

### Example 1

Human induced pluripotent stem cells (hiPSCs) were maintained in mTESR1 medium (Stem cell technologies, #5850) on growth factor reduced Matrigel (Corning, #356234) at 37° C with 5% CO2 and passaged once a week. Cells for tested for mycoplasma (Lonza, #LT07-218) contamination at least once a month.

hiPSCs were seeded at a density of 2.5-3×10⁴ cells/cm². Differentiation was induced when cells reached 80-90% confluency. To generate *MESP1*+ cardiac mesoderm, confluent hiPSCs were incubated with early mesoderm induction medium, i.e., BPEL medium (Ng *et al*, 2008) supplemented with 20 ng/mL Activin-A (Miltenyi Biotec, #130-115-012), 20 ng/mL BMP4 (R&D systems, #314-BP-010/CF), and 1.5 µmol/L CHIR99021 (Axon Medchem, #1386) for three days. *MESP1* expression was determined by RT-qPCR. Compared with hiPSCs on day 0, MESP1 expression was significantly higher on day 2 and day 3 (see Figure 1). As also determined by single cell RNA sequencing, *EOMES andMESP2* (albeit at lower levels than day 4) are also expressed on day 3 (see Figure 2). On day 3, cells were incubated with late mesoderm induction medium, i.e., BPEL medium containing 5 µmol/L XAV939 (Tocris Bioscience, #3748/10) for 24 hours that resulted in a population where a large fraction expressed ROR1+ and ROR2+. ROR1 and ROR2 expression was determined by flow cytometry. Whereas on day 3, a large majority of the early mesoderm cell population expressed either ROR1 or ROR2, a large majority of the cell fraction on day 4 expressed both ROR1 and ROR2 (see Figure 3). Next, to generate *TEX5*+ and *TEX18*+ cardiac progenitor cells that will give rise to sinoatrial node (SAN)-like cells, ROR1+ and ROR2+ mesoderm cells on day 4 were incubated with cardiac progenitor induction medium, i.e., BPEL medium containing 2.5 ng/mL BMP4, 5 µmol/L XAV939, 5 µmol/L SB431542 (Tocris, #1614), 250 nmol/L RA (Sigma, #R2625-50MG) and 250 nmol/L PD173074 (Selleck Chemicals, #1264). Resulting cells on day 6 expressed *TBX5* and *TBX18* as determined by RT-qPCR (see Figure 4). From day 6 onwards cells were incubated in SAN induction medium and refreshed every 2 - 3 days until day 20. Notable electrophysiological properties of resulting SAN-like cells in comparison with ventricular-like cardiomyocytes are a) a less negative maximum diastolic depolarization (MDP), b) faster interval and c) slower upstroke velocity. Furthermore, similar to in vivo SAN cells, SAN-like cells in vitro showed responses to ivabradine, which targets funny current, *I*_{f} and carbachol, which activates inward rectifier potassium current, *I*_{KACH} (Figure 8). We also assessed heterogeneity and composition of the cultures contained SAN-like cells, which revealed the presence of SAN-head (28%), SAN-tail (23%) and SAN-TZ (20%) like cell populations as determined by single cell RNA sequencing. A small proportion of proepicardial cells (15%) and sinus venosus-like cells (14%) were also detected. SAN-head cells expressed *TBX18*, *SHOX2*, *TBX3*, *ISL1* and were devoid of *NKX2-5.* SAN-tail cells expressed *NKX2-5* in addition to *SHOX2*, *TBX3*, *TBX3*, *ISL1* and were devoid of *TBX18.* SAN-TZ cells expressed *NKX2-*5 and *NPPA* in addition to *TBX3* and were devoid of *TBX18*, and *SHOX2.* Proepicardial cells could be identified by the expression of *WT1* and *ALDH1A2.* Sinus venosus-like cells could be identified by the expression of *TBX18*, *SHOX2*, *ISL1* and they were devoid of *TBX3* and *NKX2-5.* Expression profiles of the cell populations as assessed by single cell RNA sequencing is shown in Figure 5.

### Example 2

hiPSCs were seeded at a density of 2.5-3×10⁴ cells/cm². Differentiation was induced when cells reached 80-90% confluency. To generate *MESP1*+ cardiac mesoderm, confluent hiPSCs were incubated with early mesoderm induction medium, i.e., BPEL medium (Ng *et al*, 2008) supplemented with 20 ng/mL Activin-A (Miltenyi Biotec, #130-115-012), 20 ng/mL BMP4 (R&D systems, #314-BP-010/CF), and 1.5 µmol/L CHIR99021 (Axon Medchem, #1386) for three days. *MESP1* expression was determined by RT-qPCR. Compared with hiPSCs on day 0, MESP1 expression was significantly higher on day 2 and day 3 (see Figure 1). On day 3, cells were incubated with late mesoderm induction medium, i.e., BPEL medium containing 5 µmol/L XAV939 (Tocris Bioscience, #3748/10) for 24 hours that resulted in a population where a large fraction expressed ROR1+ and ROR2+. ROR1 and ROR2 expression was determined by flow cytometry. Whereas on day 3, a large majority of the early mesoderm cell population expressed either ROR1 or ROR2, a large majority of the cell fraction on day 4 expressed both ROR1 and ROR2 (see Figure 2). Next, to generate *TBX5*+ and *TBX18*+ cardiac progenitor cells that will give rise to sinoatrial node (SAN)-like cells, ROR1+ and ROR2+ mesoderm cells on day 4 were incubated with cardiac progenitor induction medium, i.e., BPEL medium containing 2.5 ng/mL BMP4, 5 µmol/L XAV939, 5 µmol/L SB431542 (Tocris, #1614), 250 nmol/L RA (Sigma, #R2625-50MG) and 250 nmol/L PD173074 (Selleck Chemicals, #1264). Resulting cells on day 6 expressed *TBX5* and *TBX18* as determined by RT-qPCR (see Figure 3). For enhancing the percentage of SAN-head cells, cardiac progenitor cells obtained on day 6 were incubated with SAN induction medium supplemented with medium containing 5 µmol/L XAV939 for 8 days. The resulting cell population expressed higher levels of *SHOX2*, *NTM, VSNL1* and *FLRT3* compared with the mean of the expression levels of *SHOX2*, *NTM, VSNL1* and *FLRT3* of the cell population obtained in example 1, indicating the presence of an increased proportion of SAN-head like cardiomyocytes (see Figure 6).

### Example 3

hiPSCs were seeded at a density of 2.5-3×10⁴ cells/cm². Differentiation was induced when cells reached 80-90% confluency. To generate *MESP1*+ cardiac mesoderm, confluent hiPSCs were incubated with early mesoderm induction medium, i.e., BPEL medium (Ng *et al*, 2008) supplemented with 20 ng/mL Activin-A (Miltenyi Biotec, #130-115-012), 20 ng/mL BMP4 (R&D systems, #314-BP-010/CF), and 1.5 µmol/L CHIR99021 (Axon Medchem, #1386) for three days. *MESP1* expression was determined by RT-qPCR. Compared with hiPSCs on day 0, MESP1 expression was significantly higher on day 2 and day 3 (see Figure 1). On day 3, cells were incubated with late mesoderm induction medium, i.e., BPEL medium containing 5 µmol/L XAV939 (Tocris Bioscience, #3748/10) for 24 hours that resulted in a population where a large fraction expressed ROR1+ and ROR2+. ROR1 and ROR2 expression was determined by flow cytometry. Whereas on day 3, a large majority of the early mesoderm cell population expressed either ROR1 or ROR2, a large majority of the cell fraction on day 4 expressed both ROR1 and ROR2 (see Figure 2). Next, to generate cardiac progenitor cells that will give rise to sinoatrial node (SAN) transition zone-like (SAN-TZ) cells, ROR1+ and ROR2+ mesoderm cells on day 4 were incubated with cardiac progenitor induction medium, i.e., BPEL medium containing 2.5 ng/mL BMP4, 5 µmol/L XAV939, 5 ng/mL TGFB2 (R&D Systems, #302-B2-002/CF), 250 nmol/L RA (Sigma, #R2625-50MG) and 250 nmol/L PD173074 (Selleck Chemicals, #1264). From day 6 onwards cells were incubated in SAN induction medium and refreshed every 2 - 3 days until day 20. Resulting cells expressed lower levels of *SHOX2*, *NTM*, and *FLRT3* and higher levels of *NPPA,* and *ADM* compared with the expression levels of these genes in cell population obtained in Example 1 indicating a lower fraction of SAN-head like and a higher fraction of SAN-TZ like cells (see Figure 7).

### Materials and Methods

### Key Resource Table

| **Reagent type (species) or resource** | **Designati on** | **Source or reference** | **Identifiers** |
|---|---|---|---|
| Peptide, recombin ant protein | Activin-A | Miltenyi Biotec | #130-115-012 |
| Peptide, recombin ant protein | BMP4 | R&D systems | #314-BP-010/CF |
| Chemical compoun d, drug | CHIR9902 1 | Axon Medchem | #1386 |
| Chemical compoun d, drug | XAV939 | Tocris Bioscience | #3748/10 |
| Chemical compoun d, drug | Retinoic Acid | Sigma | #R2625 |
| Chemical compoun d, drug | SB431542 | Tocris Bioscience | #1614 |
| Chemical compoun d, drug | PD173074 | Selleck Chemicals | #1264 |
| Peptide, recombin ant protein | TGFB2 | R&D systems | #302-B2-002/CF |
| Anti ROR1 | Antibody | Miltenyi Biotec | 130-119-950 |
| Anti ROR2 | Antibody | R&D Systems | MAB20641-SP |
| Software, algorithm | Seurat V3/V4 | 10.1016/j.cell.2019.05.0 31 (V3) | https:// github.com/ satijalab/ seurat/ |
| Software, algorithm | GraphPad Prism version 9.1.0 | GraphPad Software, San Diego, California USA | https://www.graphpad.com/ |

### Primer sequences used for RT-qPCR

| | | |
|---|---|---|
| *RPLP0* | SEQ ID NO:1 ATCCAGCAGGTGTTCGACAAT | SEQ ID NO:2 GCCAGGACTCGTTTGTACCC |
| *GUSB* | SEQ ID NO:3 CCTGCGTCCCACCTAGAATC | SEQ ID NO:4 ATACGGAGCCCCCTTGTCT |
| *MESP1* | SEQ ID NO:5 GAAGGGCAGGCGATGGAG | SEQ ID NO:6 CAGGTCTCCAACAGAGCCAG |
| *TBX5* | SEQ ID NO:71 ACATGGAGCTGCACAGAATG | SEQ ID NO:8 TGCTGAAAGGACTGTGGTTG |
| *TEX18* | SEQ ID NO:9 TTGCTAAAGGCTTCCGAGAC | SEQ ID NO:10 AGGTGGAGGAACTTGCATTG |
| *SHOX2* | SEQ ID NO:11 CCATAAAGGTGTTCTCATAGGGGC | SEQ ID NO:12 AACCTGAAAGGACAAGGGCG |
| *NTM* | SEQ ID NO:13 CATCATGCTATTTGGTCCAGGC | SEQ ID NO:14 GGGAAGTGGCACTCACATCA |
| *VSNL1* | SEQ ID NO:15 GGAAGCAGAATAGCAAACTGGC | SEQ ID NO:16 GAGGCGTCTCCATAAGGAAAGA |
| *FLRT3* | SEQ ID NO:17 AGACGGGGGAGGGCTATTAC | SEQ ID NO:18 GGTCAGCAGTGTTGAGGTCTT |
| *NPPA* | SEQ ID NO:19 CGATCTGCCCTCCTAAAAAGC | SEQ ID NO:20 TTGTCCTCCCTGGCTGTTATC |
| *ADM* | SEQ ID NO:21 TTGGCAGATCACTCTCTTAGCA | SEQ ID NO:22 GACATCCGCAGTTCCCTCT |

### Maintenance of hiPSC lines and differentiation to CMs

hiPSCs were maintained in mTESR1 medium (Stem cell technologies, #5850) on growth factor reduced Matrigel (Corning, #356234) at 37° C with 5% CO2 and passaged once a week. Cells for tested for mycoplasma (Lonza, #LT07-218) contamination at least once a month.

For cardiac differentiation, cells were seeded at a density of 2.5-3×10⁴ cells/cm². Differentiation was induced when cells reached 80-90% confluency using BPEL medium (Ng *et al*, 2008) supplemented with 20 ng/mL Activin-A (Miltenyi Biotec, #130-115-012), 20 ng/mL BMP4 (R&D systems, #314-BP-010/CF), and 1.5 µmol/L CHIR99021 (Axon Medchem, #1386). Three days after initiation, medium was replaced with BPEL containing 5 µmol/L XAV939 (Tocris Bioscience, #3748/10). For SANCM differentiation, 5 µmol/L XAV939, 2.5 ng/mL BMP4, 5 µmol/L SB431542 (Tocris, #1614), 250 nmol/L RA (Sigma, #R2625-50MG) and 250 nmol/L PD173074 (Selleck Chemicals, #1264) were added on day 4. Differentiation medium was replaced with BPEL medium after 48 hours (SANCM) or 96 hours (VCM) and cells refreshed every three days thereafter. To evaluate the role of canonical WNT signaling for differentiation towards SAN-head lineage, XAV939 (5 µmol/L), was added from day 6 - day 14. To evaluate the role of TGFb signaling for differentiation towards SAN-TZ lineage, TGFb2 (R&D systems, #302-B2-002/CF; 5 ng/mL), was added from day 4 - day 6.

### RT-qPCR

Total RNA of day 19 hiPSC-derived cultures was isolated using Nucleospin RNA kit (Machery Nagel, # MN740955.50) according to the manufacturer's instructions. Reverse transcription was performed using Superscript II (ThermoFisher Scientific, #18064071) with oligo dT primers (125 µmol/L). qPCR was performed on the LightCycler 2.0 Real-Time PCR system (Roche Life Science). Primer pairs were designed to span an exon-exon junction or at least one intron (Supplementary File 6). qPCR reaction mix was prepared using the LightCycler 480 SYBR Green I Master (Roche, #4887352001), primers (1 (µmol/L) and cDNA (equivalent to 10 ng RNA). Amplification of target sequences was performed using the following protocol: 5 min at 95 °C followed by 45 cycles of 10 sec at 95 °C, 20 sec at 60 °C, and 20 sec at 72 °C. Data analysis was performed using LinRegPCR program (Ruijter *et al*, 2009). For data normalization, two experimentally assessed reference genes, RPLP0 and GUSB were used.

### Single Cell Patch-Clamp

Day 16 cardiomyocytes were dissociated using 1× TryPLE Select (ThermoFisher Scientific #12563011) and plated at a density of 7.0^10³ per coverslip. After one week, cells with a smooth surface and intact membrane were chosen for measurements. Action potentials were recorded at 37°C with the amphotericin-B-perforated patch-clamp technique using a Axopatch 200B Clamp amplifier (Molecular Devices Corporation). Measurements were carried out in Tyrode's solution containing 140 mmol/L NaCl, 5.4 mmol/L KCl, 1.8 mmol/L CaCl2, 1.0 mmol/L MgCl2, 5.5 mmol/L glucose and 5.0 mmol/L HEPES. pH was adjusted to 7.4 with NaOH. Pipettes (borosilicate glass; resistance 1.5-2.5 MΩ) were filled with a solution containing 125 mmol/L potassium gluconate, 20 mmol/L KCl, 10 mmol/L NaCl, 0.4 mmol/L amphotericin-B and 10 mM HEPES, pH was adjusted to 7.2 with KOH. Signals were lowpass-filtered (cut off frequency 10 kHz) and digitized at 40 kHz. Action potentials were corrected for the estimated change in liquid junction potential (Barry & Lynch, 1991). Data acquisition and analysis were performed using custom software.

### Flow cytometry

Cells were dissociated using 1× TrypLE Select (ThermoFisher Scientific #12563011). For intracellular staining, cells were fixed and stained using the FIX & PERM kit (Thermofisher; #GAS004) according to the manufacturer's instructions. For cell surface antigens, the antibody was added to the cell suspension resuspended in a buffer containing 10% BSA (Sigma Aldrich, #A8022) and 0.5 M EDTA (ThermoFisher Scientific #15575020). A matching isotype antibody was always used as a negative control. All antibody incubations were performed for 30 minutes on ice protected from light. Acquisition was performed on FacsCanto II Cell Analyzer (Beckton Dickinson). Data was analyzed using FlowJo v10. Gates to determine the positive fraction were set using the parameters of the isotype control. Antibody information is provided in the key resource table.

### Cell sorting for single cell RNA sequencing

Single cell sequencing was performed using SORT-seq method (Muraro *et al*, 2016). Cells from one representative differentiation were collected at different stages (day 0, 4, 5, 6 and 10). At the end time point on day 19, cells from two independent differentiations were collected to ascertain reproducibility. For each time point, cells were sorted into two (D0-10, D19 SANCM^{TGFB2}) or three (D19SANCM and D19ACM) 384-well plates, each well containing an oil droplet with barcoded primers, spike-ins and dNTPs. Preparation of single-cell libraries was performed using the CEL-Seq2 protocol (Muraro *et al*, 2016; Hashimshony *et al*, 2016). Paired-end sequencing was performed on the NextSeq500 platform using 1x75 bp read length kit.

### Bioinformatic Analysis

### Reference genome annotation

Mapping was performed using BWA-MEM against the (human) genome assembly GRCh38 (hg38). Count matrices were generated using MapAndGo, filtering reads with a minimum quality score of 60 and no alternative hits. *scRNAseq data preprocessing, normalization and batch-correction, clustering, differential gene expression, cell-type identification and visualization*

Data analysis was performed using the R toolkit Seurat version 3 and 4 (Stuart *et al*, 2019). Data QC and preprocessing, dimensional reduction, clustering and differential gene expression were performed according to the standard workflow (https://satijalab.org/seurat/). Briefly, high quality single cells collected on D19 were selected according to the following parameters: gene count > 1,000 and < 9,000, mRNA molecule count < 60,000 and mitochondrial gene count < 50%. The filters for the time series dataset were set the following: gene count > 600; mRNA molecule count < 100,000; mitochondrial gene count < 50%. Next, normalization, scaling and identification of variable features (nfeatures=3000) based on variance stabilizing transformation ("vst") was performed using the SCTransform command (Hafemeister & Satija, 2019). Since technical plate-to-plate variations were observed, SCTransform data integration was performed by normalizing each dataset individually, identifying integration anchors within the datasets collected on the same timepoint and integrating the datasets. Dimensionality reduction was performed using principle component (PC) analysis and Uniform Manifold Approximation and Projection (UMAP) with the top 15 PCs (day 19 datasets, Figure 3 and Figure 7), top 20 PCs (day 0 - 19 SANCM dataset, Figure 4) and seed set to 2020. For cell clustering, a KNN (K-nearest neighbor) graph was constructed based on euclidean distance in PCA space and clusters were identified using the Louvain algorithm, as implemented in the FindNeighbors and FindClusters command. Identified clusters were then visualized in a UMAP using the DimPlot command. For differential expression testing and visualization, LogNormalization was performed according to the standard workflow on the uncorrected dataset and differential gene expression was determined using Wilcoxon rank sum test. Differentially expressed gene lists show genes, which are expressed in at least 25% in either of the two fractions of cells and limited to genes, which are differentially expressed (on average) by at least 0.25-fold (log-scale) between the two compared cell fractions. Cell type specific marker genes were used to annotate cell clusters. VlnPlot, FeaturePlot and DoHeatMap commands were used to visualize gene expression.

### Statistical analysis

Statistical analysis was carried out in GraphPad Prism version 9.1.0 for Windows GraphPad Software, San Diego, California USA, www.graphpad.com. Data were represented as mean ± standard error of the mean (s.e.m.). Non-parametric tests were performed in all cases. Number of samples (n) and the method used to test statistical significance are stated in each figure legend. P < 0.05 was considered statistically significant.

## Claims

1. A method of generating a population of cardiac progenitor cells from MESP1 mesoderm cells comprising:
a) incubating a MESP1 mesoderm cell in a late mesoderm induction medium comprising a WNT inhibitor for a sufficient period of time to generate a ROR1+ and ROR2+ mesoderm cell,
b) incubating said ROR1+ and ROR2+ mesoderm cell in a cardiac progenitor induction medium comprising a BMP ligand, a WNT inhibitor, retinoic acid, a TGF-β inhibitor or TGF-β, and an FGF inhibitor for a sufficient period of time to generate a cardiac progenitor cell expressing TBX5 and TBX18.

2. The method of claim 1, wherein said cardiac progenitor induction medium comprises a BMP ligand, a WNT inhibitor, retinoic acid, an FGF inhibitor and TGF-β.

3. The method of claim 1, wherein said cardiac progenitor induction medium comprises a BMP ligand, a WNT inhibitor, retinoic acid, an FGF inhibitor and TGF-β inhibitor.

4. The method of any of the above claims, wherein said MESP1 mesoderm cell is prepared by incubating a pluripotent stem cell (PSC) in an early mesoderm induction medium comprising an activator of WNT signaling and optionally one or more members selected from a BMP ligand, a nodal signaling protein for a sufficient period of time to generate MESP1 mesoderm cells expressing EOMES and MESP1 and preferably MESP2.

5. A method for generating a population of Sinoatrial node (SAN)-like cells having SAN head characteristics from MESP1 mesoderm cells, wherein a cardiac progenitor cell is produced according to claim 3, said method further comprising incubating said cardiac progenitor cell in a SAN induction medium comprising a WNT inhibitor for a sufficient period of time to generate a population of SAN-like cells which express SHOX2, TBX18 and FLRT3 and do not substantially express NKX2-5 and NPPA.

6. A method for generating a heterogeneous population of Sinoatrial node (SAN)-like cells comprising subpopulations of SAN-like cells having SAN-head, SAN-tail and SAN-TZ characteristics from MESP1 mesoderm cells, wherein a cardiac progenitor cell is produced according to claim 3, said method further comprising incubating said cardiac progenitor cell in a SAN induction medium without a WNT inhibitor for a sufficient period of time to generate a population of SAN-like cells, wherein:
said SAN like cells having SAN-tail characteristics express ISL1, SHOX2, TBX3 and NKX2-5 and do not substantially express TBX18 and NPPA,
said SAN-like cells having SAN-head characteristics express SHOX2, TBX18 and FLRT3 and do not substantially express NKX2-5 and NPPA, and
said SAN-like cells having SAN-TZ characteristics express NPPA, NKX2.5 and TBX3 and do not substantially express TBX18 and SHOX2.

7. A method for generating a population of SAN-like cells having SAN-TZ characteristics from MESP1 mesoderm cells, wherein a cardiac progenitor cell is produced according to the method according to any of claims 1-4, and wherein said cardiac progenitor cell is further cultured in a SAN induction medium without a WNT inhibitor for a sufficient period of time to generate a population of SAN-like cells, wherein at least a proportion of SAN-like cells express NPPA, NKX2.5 and TBX3 and do not substantially express TBX18 and SHOX2.

8. The method according to claim 7, wherein said proportion is at least 5, 10, 20 or 25% of the total population.

9. The method according to any of claim 5-8, wherein the SAN-like cells exhibit electrophysiological properties of SAN cells.

10. The method according to claim 9, wherein the electrophysiological properties exhibited by SAN-like cells comprise exhibition of HCN dependent funny current.

11. A method of identifying a candidate drug comprising:
a. generating a population of SAN-like cells according to a method of any of claims 5-10;
b. contacting the SAN-like cells with a candidate test drug;
c. measuring the beat rate, action potential characteristics and/or ion currents of the SAN-like cells;
d. comparing the beat rate, the action potential characteristics and/or ion currents of the SAN-like cells to a control SAN-like cells not treated with the candidate test drug; and
e. selecting the candidate test drug which modulates the beat rate and/or action potential compared to the control cell as the candidate drug.

12. The method according to claim 11, wherein said population of cells comprises at least 5, 10, or 20% of SAN-like cells which express NPPA, NKX2.5 and TBX3 and do not substantially express TBX18 and SHOX2 or said population of cells comprises at least at least 5, 10, or 20% of SAN-like cells which express NKX2-5, SHOX2, TBX3, TBX3, ISL1 and do not substantially express TBX18.

13. An isolated SAN cell or a SAN-like cell which has the following characteristics:
expresses NPPA, NKX2.5 and TBX3 and does not substantially express TBX18 and SHOX2,
or
expresses NKX2-5, SHOX2, TBX3, TBX3, ISL1 and does not substantially express TBX18.

14. An isolated SAN cell or a SAN-like cell of claim 13, for use in a medical treatment of a patient, preferably for the treatment of bradycardia or nodal dysfunction.

15. Use of an isolated SAN cell or a SAN-like cell according to claim 14 in a drug discovery method.
